# EUROPÄISCHE PATENTANMELDUNG

(11) **EP 3 453 324 A1**
(43) Veröffentlichungstag der Anmeldung: **13.03.2019**
(21) Anmeldenummer: 17189906.5
(22) Anmeldetag: 07.09.2017
(51) Int. Cl.: A61B 5/055

(54) **MAGNETRESONANZVORRICHTUNG MIT EINER PATIENTENLAGERUNGSVORRICHTUNG MIT EINER KIPPEINHEIT**

(71) Anmelder: Siemens Healthcare GmbH, 91052 Erlangen (DE)
(72) Erfinder: Feiweier, Thorsten, 91099 Poxdorf (DE)

(57) **Zusammenfassung**

Die Erfindung geht aus von einer Magnetresonanzvorrichtung mit einer Magneteinheit, einen Patientenaufnahmebereich, welcher Patientenaufnahmebereich zumindest teilweise von der Magneteinheit umgeben ist, und eine Patientenlagerungsvorrichtung mit einem innerhalb des Patientenaufnahmebereichs bewegbaren Patiententisch, wobei die Patientenlagerungsvorrichtung eine Kippeinheit mit einer Kippachse aufweist und der Patiententisch um die Kippachse kippbar gelagert ist.

## Beschreibung

Die vorliegende Erfindung betrifft eine Magnetresonanzvorrichtung mit einer Magneteinheit, einen Patientenaufnahmebereich, welcher Patientenaufnahmebereich zumindest teilweise von der Magneteinheit umgeben ist, und eine Patientenlagerungsvorrichtung mit einem innerhalb des Patientenaufnahmebereichs bewegbaren Patiententisch.

Patientenlagerungsvorrichtung von Magnetresonanzvorrichtung weisen einen Patiententisch auf, mit dem der Patient in den Patientenaufnahmebereich für eine Magnetresonanzuntersuchung gefahren werden kann. Hierzu weist die Patientenlagerungsvorrichtung gewöhnlich eine Verschiebeeinheit, insbesondere eine horizontale Verschiebeeinheit, auf. Derartige Patientenlagerungsvorrichtungen sind beispielsweise aus DE 10 2014 218 514 A1, DE 10 2014 214 993 A1 und DE 10 2014 208 924 A1 bekannt.

Der vorliegenden Erfindung liegt insbesondere die Aufgabe zugrunde, eine erweiterte Positionierung eines Patienten für eine Magnetresonanzuntersuchung zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer Magnetresonanzvorrichtung mit einer Magneteinheit, einen Patientenaufnahmebereich, welcher Patientenaufnahmebereich zumindest teilweise von der Magneteinheit umgeben ist, und eine Patientenlagerungsvorrichtung mit einem innerhalb des Patientenaufnahmebereichs bewegbaren Patiententisch.

Es wird vorgeschlagen, dass die Patientenlagerungsvorrichtung eine Kippeinheit mit einer Kippachse aufweist und der Patiententisch um die eine Kippachse kippbar gelagert ist.

In diesem Zusammenhang soll unter einem innerhalb des Patientenaufnahmebereichs bewegbaren Patiententisch insbesondere ein Patiententisch verstanden werden, der in Richtung einer Einführbewegung in den Patientenaufnahmebereich bewegbar ausgebildet ist. Zur Bewegung des Patiententischs in Richtung der Einführbewegung weist die Patientenlagerungsvorrichtung bevorzugt eine Verschiebeeinheit, insbesondere eine horizontale Verschiebeeinheit, auf. Die horizontale Verschiebeeinheit umfasst dabei typischerweise Gleitlagerelemente, wie beispielsweise Gleitschienen, die in horizontaler Richtung innerhalb des Patientenaufnahmebereichs angeordnet sind. In diesen Gleitlagerelemente, insbesondere den Gleitschienen, ist der Patiententisch in horizontaler Richtung bewegbar gelagert.

Vorzugsweise ist die Richtung der Einführbewegung parallel zu einer horizontalen Richtung und/oder einer Längserstreckung des Patientenaufnahmebereichs ausgebildet. Zudem ist die Richtung der Einführbewegung auch parallel zu einer Richtung einer Längserstreckung des Patiententischs ausgebildet, sofern der Patiententisch einen Neigungswinkel von 0° aufweist und somit nicht gekippt ist. Mittels des Patiententischs wird ein Patient für eine Magnetresonanzuntersuchung innerhalb des Patientenaufnahmebereichs in horizontaler Richtung positioniert. Insbesondere wird derart ein zu untersuchender Bereich des Patienten innerhalb eines Erfassungsbereichs und/oder eines Fields of View (FoV) des Patientenaufnahmebereichs für die anstehende Magnetresonanzuntersuchung positioniert.

Die Patientenlagerungsvorrichtung umfasst neben der Verschiebeeinheit, insbesondere der horizontalen Verschiebeeinheit, die Kippeinheit mit einer Kippachse, um die der Patiententisch kippbar gelagert ist. Vorzugsweise ist die Kippachse im Wesentlichen quer zur Richtung der Einführbewegung ausgerichtet. Die Kippeinheit, insbesondere Kippachse, kann dabei in die Verschiebeeinheit integriert sein oder auch eine separate Einheit zur Verschiebeeinheit bilden.

Mittels der Kippeinheit kann ein Kippwinkel und/oder ein Neigungswinkel des Patiententischs eingestellt werden. Der Kippwinkel und/oder der Neigungswinkel ist bevorzugt abhängig von einem Teilbereich des Patiententischs, der in Richtung der Einführbewegung nach der Kippachse innerhalb des Patientenaufnahmebereichs angeordnet, um derart eine Kollision des Patiententischs mit einer den Patientenaufnahmebereich umgebenden Gehäusewand zu verhindern. Ein Neigungswinkel und/oder Kippwinkel beträgt dabei maximal 10°. Besonders vorteilhaft beträgt ein Neigungswinkel und/oder Kippwinkel maximal 12°. Besonders vorteilhaft beträgt ein Neigungswinkel und/oder Kippwinkel maximal 14°. Besonders vorteilhaft beträgt ein Neigungswinkel und/oder Kippwinkel maximal 16°. Besonders vorteilhaft beträgt ein Neigungswinkel und/oder Kippwinkel maximal 18°. Besonders vorteilhaft beträgt ein Neigungswinkel und/oder Kippwinkel maximal 20°.

Ein Vorgang des Kippens des Patiententischs um die Kippachse, insbesondere um den Kippwinkel und/oder den Neigungswinkel bezüglich einer Richtung einer Einführbewegung, kann zudem pneumatisch erfolgen, wobei hierzu die Kippeinheit 29 eine pneumatische Kippeinheit umfassen kann. Zudem sind weitere Einstellungsmöglichkeiten, wie insbesondere eine elektrische Einstellung und/oder eine mechanische Einstellung des Kippwinkels und/oder Neigungswinkels des Patiententischs jederzeit denkbar.

Die Erfindung ermöglicht es, dass ein Benutzer, insbesondere ein die Magnetresonanzuntersuchung betreuendes medizinisches Bedienpersonal, eine vorteilhafte Lagerung und/oder Positionierung des zu untersuchenden Bereichs des Patienten innerhalb des Patientenaufnahmebereichs vornehmen kann. Insbesondere kann der Benutzer neben einer Positionierung eines zu untersuchenden Bereichs des Patienten in horizontaler Richtung auch eine erweiterte Positionierung durch Neigung des Patiententischs und/oder durch Einstellung eines Kippwinkels des Patiententischs durchführen.

Dies kann auch eine vorteilhafte Optimierung einer Bildqualität von erfassten Magnetresonanzdaten zur Folge haben. Beispielsweise können für unterschiedliche zu untersuchende Körperregionen des Patienten auch unterschiedliche Neigungen und/oder Kipppositionen, insbesondere Kippwinkel, des Patiententischs eingestellt werden. Auch aufgrund einer Neigung und/oder einer gekippten Position des Patiententischs können spezielle Magnetresonanzuntersuchungen, wie beispielsweise eine Knie-Magnetresonanzuntersuchung oder eine Fuß-Magnetresonanzuntersuchung, des Patienten unter Belastung des entsprechenden zu untersuchenden Körperbereichs erfolgen.

Des Weiteren können derart auch Patienten, bei denen während einer Magnetresonanzuntersuchung Kreislaufprobleme auftreten, in einer gekippten Position, bei der vorteilhaft der Kopfbereich des Patienten erhöht gegenüber dem Fußbereich gelagert ist, positioniert werden und damit ein Wohlbefinden des Patienten während der Magnetresonanzuntersuchung erhöht werden. Zudem kann für einen Patienten aufgrund der gekippten Lage und/oder Position des Patiententischs ein größeres Sichtfeld während der Magnetresonanzuntersuchung ermöglicht werden gegenüber einer waagrechten Lagerung des Patiententischs und/oder des Patienten. Die gekippte Position des Patiententischs ermöglicht beispielsweise bei einer Positionierung eines Kopfbereichs innerhalb des Patientenaufnahmebereichs, dass sich das Sichtfeld des Patienten auch auf Bereich außerhalb des Patientenaufnahmebereichs erstrecken kann. Bei einer horizontalen Lagerung des Patienten innerhalb des Patientenaufnahmebereichs erstreckt sich das Sichtfeld des Patienten lediglich nur auf die den Patientenaufnahmebereich umgebende Gehäusewand. Dies kann insbesondere bei Patienten, die zu Klaustrophobie neigen, zu einer Beruhigung der Situation während der Magnetresonanzuntersuchung führen und damit eine Patientenkomfort und/oder ein Wohlbefinden des Patienten steigern.

In einer vorteilhaften Weiterbildung der Erfindung kann es vorgesehen sein, dass die Kippachse quer zu einer Längserstreckung des Patiententischs ausgerichtet ist. Die Kippachse ist bevorzugt senkrecht zur Längserstreckung des Patiententischs und/oder senkrecht zur Einführbewegung des Patiententischs in den Patientenaufnahmebereich ausgerichtet. Dies ermöglicht eine konstruktiv einfache Integration der Kippeinheit, insbesondere der Kippachse, innerhalb der Patientenlagerungsvorrichtung.

Darüber hinaus kann es vorgesehen sein, dass eine Position des Patiententischs in Richtung seiner Längserstreckung bezüglich der Kippachse einstellbar und/oder festlegbar ist, wobei der Patiententisch in der eingestellten Position um die Kippachse kippbar ist. Das Einstellen und/oder das Festlegen der Position des Patiententischs kann dabei manuell durch einen Benutzer oder auch automatisch mittels einer Patiententischsteuerungseinheit erfolgen. Diese Ausgestaltung der Erfindung ermöglicht eine individuelle Einstellung und/oder Festlegung der Position des Patiententischs, die insbesondere an Parameter für die anstehende Magnetresonanzuntersuchung angepasst werden kann.

Besonders vorteilhaft ist hierbei eine Einstellung und/oder Festlegung der Position des Patiententischs in Richtung seiner Längserstreckung bezüglich der Kippachse und/oder eine Einstellung eines Kippwinkels des Patiententischs um die Kippachse abhängig von zumindest einem Patientenparameter und/oder zumindest einem Untersuchungsparameter. Der zumindest eine Patientenparameter kann dabei einen Körpergröße des Patienten und/oder einen Gesundheitszustand des Patienten und/oder einen aktuelle Stresswert des Patienten und/oder eine Information, ob der Patient klaustrophobisch veranlagt ist, usw. umfassen. Der zumindest eine Untersuchungsparameter kann eine Bildqualität und/oder eine klinische und/oder diagnostische Fragestellung und/oder eine zu untersuchende Körperregion usw. umfassen.

In einer bevorzugten Ausgestaltung der Erfindung kann es vorgesehen sein, dass die Patientenlagerungsvorrichtung eine Verschiebeeinheit aufweist und die Kippeinheit, insbesondere die Kippachse der Kippeinheit, innerhalb der Verschiebeeinheit angeordnet ist. Dies ermöglicht eine besonders kompakte und platzsparende Bauweise der Patientenlagerungsvorrichtung. Die Verschiebeeinheit umfasst bevorzugt eine horizontale Verschiebeeinheit, die zu einem Verschieben des Patiententischs in horizontaler Richtung innerhalb des Patientenaufnahmebereichs ausgelegt ist.

Weist der Patientenaufnahmebereich einen Durchmesser von mindestens 70 cm auf, kann somit besonders vorteilhaft ein Raum zum Kippen und/oder Neigen des Patiententischs innerhalb des Patientenaufnahmebereichs zur Verfügung gestellt werden. Besonders vorteilhaft jedoch weist der Patientenaufnahmebereich einen Durchmesser von mindestens 75 cm auf. Besonders vorteilhaft jedoch weist der Patientenaufnahmebereich einen Durchmesser von mindestens 80 cm auf. Besonders vorteilhaft jedoch weist der Patientenaufnahmebereich einen Durchmesser von mindestens 85 cm auf.

Des Weiteren geht die Erfindung aus von einem Verfahren zu einem Bewegen und/oder Positionieren eines Patiententischs einer Patientenlagerungsvorrichtung einer Magnetresonanzvorrichtung nach einem der vorhergehenden Ansprüche. Hierbei wird der Patiententisch in einem ersten Positionierungsschritt zunächst horizontal verschoben und in einem zweiten Positionierungsschritt mittels der Kippeinheit um die Kippachse gekippt.

Das erfindungsgemäße Verfahren ermöglicht es, dass ein Benutzer, insbesondere ein die Magnetresonanzuntersuchung betreuendes medizinisches Bedienpersonal, eine vorteilhafte Lagerung und/oder Positionierung des zu untersuchenden Bereichs des Patienten innerhalb des Patientenaufnahmebereichs vornehmen kann. Insbesondere kann der Benutzer neben einer Positionierung eines zu untersuchenden Bereichs des Patienten in horizontaler Richtung auch eine erweiterte Positionierung durch Neigung des Patiententischs und/oder durch Einstellung eines Kippwinkels des Patiententischs durchführen.

Dies kann auch eine vorteilhafte Optimierung einer Bildqualität von erfassten Magnetresonanzdaten zur Folge haben. Beispielsweise können für unterschiedliche zu untersuchende Körperregionen des Patienten auch unterschiedliche Neigungen und/oder Kipppositionen, insbesondere Kippwinkel, des Patiententischs eingestellt werden.

Des Weiteren können derart auch Patienten, bei denen während einer Magnetresonanzuntersuchung Kreislaufprobleme auftreten, in einer gekippten Position, bei der vorteilhaft der Kopfbereich des Patienten erhöht gegenüber dem Fußbereich gelagert ist, positioniert werden und damit ein Wohlbefinden des Patienten während der Magnetresonanzuntersuchung erhöht werden. Zudem kann für einen Patienten aufgrund der gekippten Lage und/oder Position des Patiententischs ein größeres Sichtfeld während der Magnetresonanzuntersuchung ermöglicht werden gegenüber einer waagrechten Lagerung des Patiententischs und/oder des Patienten.

Die Vorteile des erfindungsgemäßen Verfahrens zu einem Bewegen und/oder Positionieren eines Patiententischs entsprechen im Wesentlichen den Vorteilen der erfindungsgemäßen Magnetresonanzvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung mit einem kippbaren Patiententisch in einer schematischen Darstellung und
- Fig. 2: ein Verfahren zu einem Bewegen eines kippbaren Patiententischs.

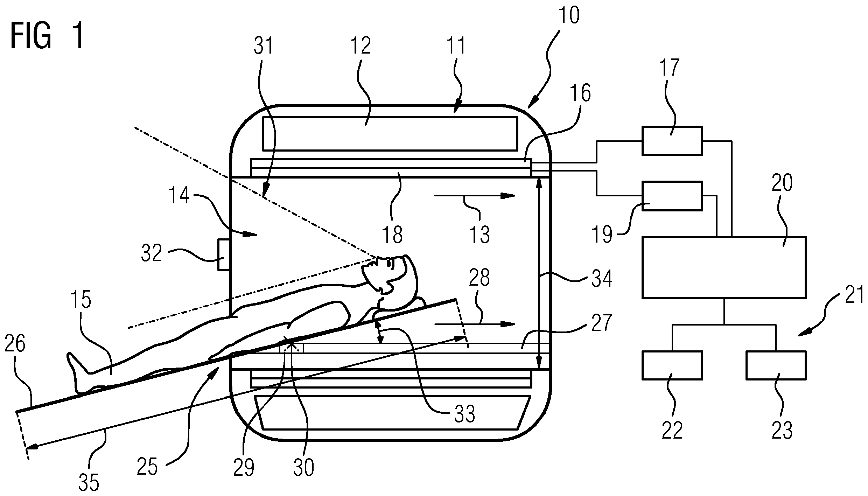

In Fig. 1 ist eine Magnetresonanzvorrichtung 10 schematisch dargestellt. Die Magnetresonanzvorrichtung 10 umfasst eine Magneteinheit 11, die einen supraleitenden Hauptmagneten 12 zu einem Erzeugen eines starken und insbesondere konstanten Hauptmagnetfelds 13 umfasst. Zudem weist die Magnetresonanzvorrichtung 10 einen Patientenaufnahmebereich 14 auf zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patientenaufnahmebereich 14 weist einen Durchmesser 34 von mindestens 70 cm auf. Besonders vorteilhaft weist der Patientenaufnahmebereich 14 einen Durchmesser 34 von mindestens 75 cm auf. Besonders vorteilhaft weist der Patientenaufnahmebereich 14 einen Durchmesser 34 von mindestens 80 cm auf. Besonders vorteilhaft weist der Patientenaufnahmebereich 14 einen Durchmesser 34 von mindestens 85 cm auf.

Die Magneteinheit 11 weist weiterhin eine Gradientenspuleneinheit 16 zu einer Erzeugung von Magnetfeldgradienten auf, die für eine Ortskodierung während einer Bildgebung verwendet werden. Die Gradientenspuleneinheit 16 wird mittels einer Gradientensteuereinheit 17 der Magnetresonanzvorrichtung 10 gesteuert. Die Magneteinheit 11 umfasst weiterhin eine Hochfrequenzantenneneinheit 18 zu einer Anregung einer Polarisation, die sich in dem von dem Hauptmagneten 12 erzeugten Hauptmagnetfeld 13 einstellt. Die Hochfrequenzantenneneinheit 18 wird von einer Hochfrequenzantennensteuereinheit 19 der Magnetresonanzvorrichtung 10 gesteuert und strahlt hochfrequente Magnetresonanzsequenzen in einen Untersuchungsraum, der im Wesentlichen von einem Patientenaufnahmebereich 14 der Magnetresonanzvorrichtung 10 gebildet ist, ein.

Zu einer Steuerung des Hauptmagneten 12, der Gradientensteuereinheit 16 und zur Steuerung der Hochfrequenzantennensteuereinheit 18 weist die Magnetresonanzvorrichtung 10 eine Systemsteuereinheit 20 auf. Die Systemsteuereinheit 20 steuert zentral die Magnetresonanzvorrichtung 10, wie beispielsweise das Durchführen einer vorbestimmten bildgebenden Gradientenechosequenz. Zudem umfasst die Systemsteuereinheit 20 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 21, die mit der Systemsteuereinheit 20 verbunden ist. Steuerinformationen wie beispielsweise Bildgebungsparameter, sowie rekonstruierte Magnetresonanzbilder können auf einer Anzeigeeinheit 22, beispielsweise auf zumindest einem Monitor, der Benutzerschnittstelle 21 für ein medizinisches Bedienpersonal angezeigt werden. Weiterhin weist die Benutzerschnittstelle 21 eine Eingabeeinheit 23 auf, mittels der Informationen und/oder Parameter während eines Messvorgangs von dem medizinischen Bedienpersonal eingegeben werden können.

Für eine Positionierung und/oder einen Transport des Patienten 15 innerhalb des Patientenaufnahmebereichs 14 weist die Magnetresonanzvorrichtung 10 eine Patientenlagerungsvorrichtung 25 auf. Die Patientenlagerungsvorrichtung 25 weist einen innerhalb des Patientenaufnahmebereichs 14 bewegbar ausgestalteten Patiententisch 26 auf. Für eine horizontale Bewegung und/oder Verschiebung des Patiententischs 26 in Richtung 28 einer Einführbewegung, insbesondere einer horizontalen Einführbewegung, weist die Patientenlagerungsvorrichtung 25 eine Verschiebeeinheit 27, insbesondere eine horizontale Verschiebeeinheit 27, auf. Mittels der Verschiebeeinheit 27, insbesondere der horizontalen Verschiebeeinheit 27, wird der Patiententisch 26 in Richtung einer Längserstreckung des zylinderförmigen Patientenaufnahmebereichs 14 verschoben. Die Richtung der Längserstreckung des Patientenaufnahmebereichs 14 und die Richtung 28 der Einführbewegung des Patiententischs 26 sind dabei parallel zu einander. Zudem ist die Richtung 28 der Einführbewegung des Patiententischs 26 auch parallel zu einer Richtung einer Längserstreckung 35 des Patiententischs 26, sofern dieser in einer nicht gekippten Position ist.

Des Weiteren umfasst die Patientenlagerungsvorrichtung 25 eine Kippeinheit 29 mit einer Kippachse 30, wobei der Patiententisch 26 um die Kippachse 30 kippbar gelagert ist. Die Kippeinheit 29, insbesondere die Kippachse 30, ist innerhalb der Verschiebeeinheit 27, insbesondere der horizontalen Verschiebeeinheit 27, integriert. Die Kippachse 30 ist quer, insbesondere senkrecht, zur Längserstreckung 35 des Patiententischs 26 ausgerichtet. Zudem ist die Kippachse 30 auch senkrecht zur Verschiebeeinheit 27 und/oder zur Richtung 28 der Einführbewegung des Patiententischs 26 ausgerichtet.

Die Kippeinheit 29 ist in einem vorderen Bereich 31 des Patientenaufnahmebereichs 14 angeordnet, wobei der vordere Bereich 31 einen Einführbereich zu einem Einführen des Patiententischs 26 in den Patientenaufnahmebereich 14 aufweist. Bei einem Einführen und/oder Einfahren des Patiententischs 26 in den Patientenaufnahmebereich 14 fährt der Patiententisch 26 zuerst in den vorderen Bereich 31 und/oder in den Einführbereich ein. Der vordere Bereich 31 und/oder Einführbereich ist somit in Richtung 28 der Einführbewegung einem Field of View und/oder einem Erfassungsbereich der Magneteinheit innerhalb des Patientenaufnahmebereichs 14 11 vorgelagert.

Für eine Einstellung einer Neigung und/oder eine Kippposition des Patiententischs 26 wird zunächst der Patiententisch 26 in Richtung 28 der Einführbewegung mittels der horizontalen Verschiebeeinheit 27 positioniert. Anschließend erfolgt mittels der Kippeinheit 29 ein Einstellen einer Neigung und/oder einer Kippposition des Patiententischs 26.

Die Position des Patiententischs 26, in der dieser mittels der Kippeinheit um die Kippachse 30 drehbar ist, kann von einem Benutzer eingestellt und/oder festgelegt werden. Alternativ hierzu ist auch möglich, dass die Position des Patiententischs 26, in der dieser mittels der Kippeinheit 29 um die Kippachse 30 drehbar ist, automatisch von einer Patiententischsteuerungseinheit 32 eingestellt und/oder festgelegt werden kann. Die Kippeinheit 29 ist bevorzugt derart ausgestaltet, dass unterschiedliche Positionen des Patiententischs 26 in Richtung seiner Längserstreckung 35 bezüglich der Kippachse 30 eingestellt werden können. Ein Kippen des Patiententischs 26 um die Kippachse 30 und/oder um den Kippwinkel 33 und/oder den Neigungswinkel bezüglich der Richtung 28 kann zudem pneumatisch erfolgen, wobei hierzu die Kippeinheit 29 eine pneumatische Kippeinheit umfassen kann. Zudem sind weitere, dem Fachmann als sinnvoll erscheinende Ausgestaltungen der Kippeinheit 29 zum Kippen des Patiententischs 26 um die Kippachse 30 jederzeit denkbar.

Das Einstellen und/oder Festlegen der Position der Patiententischs 26 in Richtung seiner Längserstreckung 35 bezüglich der Kippachse 30 kann dabei abhängig sein von zumindest einem Patientenparameter und/oder zumindest einem Untersuchungsparameter. Der zumindest eine Patientenparameter kann dabei einen Körpergröße des Patienten 15 und/oder einen Gesundheitszustand des Patienten 15 und/oder einen aktuelle Stresswert des Patienten 15 und/oder eine Information, ob der Patient 15 klaustrophobisch veranlagt ist, usw. umfassen. Der zumindest eine Untersuchungsparameter kann eine Bildqualität und/oder eine klinische und/oder diagnostische Fragestellung und/oder eine zu untersuchende Körperregion usw. umfassen.

Zudem kann auch eine Einstellung und/oder Festlegung eines Neigungswinkels und/oder eines Kippwinkels 33 des Patiententischs 26 abhängig sein von zumindest einem Patientenparameter und/oder zumindest einem Untersuchungsparameter. Beispielsweise können für eine Magnetresonanzuntersuchung der Wirbelsäule eines Patienten 15 für unterschiedliche Bereiche der Wirbelsäule auch unterschiedliche Positionen und/oder Einstellung zur Neigung und/oder Kippposition des Patiententischs 26 eingestellt werden, um somit für jeden Bereich der Wirbelsäule optimale Bilddaten zur Verfügung zu stellen.

Der Kippwinkel und/oder der Neigungswinkel kann auch abhängig von einem Teilbereich des Patiententischs 26, der in Richtung 28 der Einführbewegung nach der Kippachse 30 innerhalb des Patientenaufnahmebereichs 14 angeordnet ist, sein, um derart eine Kollision des Patiententischs 26 mit einer den Patientenaufnahmebereich 14 umgebenden Gehäusewand zu verhindern. Ein Neigungswinkel und/oder Kippwinkel beträgt dabei maximal 10°. Besonders vorteilhaft beträgt ein Neigungswinkel und/oder Kippwinkel maximal 12°. Besonders vorteilhaft beträgt ein Neigungswinkel und/oder Kippwinkel maximal 14°. Besonders vorteilhaft beträgt ein Neigungswinkel und/oder Kippwinkel maximal 16°. Besonders vorteilhaft beträgt ein Neigungswinkel und/oder Kippwinkel maximal 18°. Besonders vorteilhaft beträgt ein Neigungswinkel und/oder Kippwinkel maximal 20°.

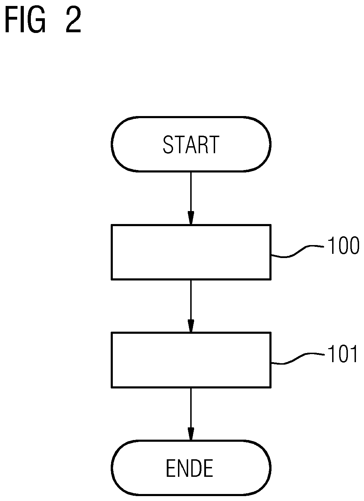

In Fig. 2 ist ein Verfahren zu einem Bewegen und/oder Positionieren des Patiententischs 26 der Patientenlagerungsvorrichtung 25 dargestellt. In einem ersten Positionierungsschritt 100 erfolgt eine horizontale Verschiebung und/oder Positionierung des Patiententischs 26 mittels der Verschiebeeinheit 27, insbesondere der horizontalen Verschiebeeinheit 27. Bevorzugt wird derart der zu untersuchende Bereich des Patienten 15 innerhalb des Fields of View der Magneteinheit 11 angeordnet. Anschließend erfolgt in einem zweiten Positionierungsschritt 101 ein Einstellen einer Kippposition und/oder einer Neigung des Patiententischs 26 um die Kippachse 30 mittels der Kippeinheit 29. Während es zweiten Positionierungsschritts 101 kann der Patiententisch 26 in Richtung 28 der Einführbewegung des Patiententischs 26 arretiert sein, so dass ein unerwünschtes Bewegen in Richtung der Längserstreckung des Patiententischs 16 aufgrund des Kippvorgangs verhindert werden kann. Die Arretierung des Patiententischs 26 kann dabei bevorzugt mittels der Verschiebeeinheit 27, insbesondere der horizontalen Verschiebeeinheit 27, erfolgen.

Obwohl die Erfindung im Detail durch die bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung (10) mit einer Magneteinheit (11), einen Patientenaufnahmebereich (14), welcher Patientenaufnahmebereich (14) zumindest teilweise von der Magneteinheit (11) umgeben ist, und eine Patientenlagerungsvorrichtung (25) mit einem innerhalb des Patientenaufnahmebereichs (14) bewegbaren Patiententisch (26),
**dadurch gekennzeichnet, dass** die Patientenlagerungsvorrichtung (25) eine Kippeinheit (29) mit einer Kippachse (30) aufweist und der Patiententisch (26) um die Kippachse (30) kippbar gelagert ist.

2. Magnetresonanzvorrichtung (10) nach Anspruch 1,
**dadurch gekennzeichnet, dass** die Kippachse (30) quer zu einer Längserstreckung (35) des Patiententischs (26) ausgerichtet ist.

3. Magnetresonanzvorrichtung (10) nach einem der vorhergehenden Ansprüche,
**dadurch gekennzeichnet, dass** eine Position des Patiententischs (26) in Richtung seiner Längserstreckung (35) bezüglich der Kippachse (30) einstellbar ist, wobei der Patiententisch (26) in der eingestellten Position um die Kippachse (30) kippbar ist.

4. Magnetresonanzvorrichtung (10) nach Anspruch 3,
**dadurch gekennzeichnet, dass** eine Einstellung der Position der Patiententischs (26) in Richtung seiner Längserstreckung (35) bezüglich der Kippachse (30) und/oder eine Einstellung eines Kippwinkels (33) des Patiententischs (26) um die Kippachse (30) abhängig ist von zumindest einem Patientenparameter.

5. Magnetresonanzvorrichtung (10) nach einem der Ansprüche 3 oder 4,
**dadurch gekennzeichnet, dass** eine Einstellung der Position der Patiententischs (26) in Richtung seiner Längserstreckung (25) bezüglich der Kippachse (30) und/oder eine Einstellung eines Kippwinkels (33) des Patiententischs (26) um die Kippachse (30) abhängig ist von zumindest einem Untersuchungsparameter.

6. Magnetresonanzvorrichtung (10) nach einem der vorhergehenden Ansprüche,
**dadurch gekennzeichnet, dass** die Patientenlagerungsvorrichtung (25) eine Verschiebeeinheit (27) aufweist und die Kippeinheit (29) innerhalb der Verschiebeeinheit (27) angeordnet ist.

7. Magnetresonanzvorrichtung (10) nach einem der vorhergehenden Ansprüche,
**dadurch gekennzeichnet, dass** der Patientenaufnahmebereich (14) einen Durchmesser (34) von mindestens 70 cm aufweist.

8. Verfahren zu einem Bewegen und/oder Positionieren eines Patiententischs (26) einer Patientenlagerungsvorrichtung (25) einer Magnetresonanzvorrichtung (10) nach einem der vorhergehenden Ansprüche, wobei der Patiententisch (26) in einem ersten Positionierungsschritt (100) horizontal verschoben wird und in einem zweiten Positionierungsschritt (101) mittels der Kippeinheit (29) um die Kippachse (30) gekippt wird.
